# EUROPÄISCHE PATENTSCHRIFT

(11) **EP 0 703 281 B1**
(45) Veröffentlichungstag und Bekanntmachung des Hinweises auf die Patenterteilung: **01.03.2000**
(21) Anmeldenummer: 95810570.2
(22) Anmeldetag: 14.09.1995
(51) Int. Cl.: C09B 47/10, C07D 487/22, G11B 7/24

(54) **Verfahren zur Herstellung von bromierten, Alkoxy-substituierten Metall-Phthalocyaninen**
Process for the manufacture of brominated, alkoxy substituted metal phthalocyanine
Procédé de fabrication de phtalocyanine de métal bromées et substituées par des alcoxy

(30) Priorität: 23.09.1994 CH 290794; 30.11.1994 CH 361194
(43) Veröffentlichungstag der Anmeldung: 27.03.1996
(73) Patentinhaber: Ciba Specialty Chemicals Holding Inc., 4057 Basel (CH)
(72) Erfinder: Wolleb, Heinz, Dr., CH-1723 Marly (CH)

(56) Entgegenhaltungen:
- EP-A- 0 513 370
- GB-A- 2 068 993
- DATABASE WPI Section Ch, Week 9209 Derwent Publications Ltd., London, GB; Class E12, AN 92-069967 & JP-A-04 015 264 ( MITSUI TOATSU CHEM INC) , 20.Januar 1992

## Beschreibung

Die Erfindung betrifft ein Verfahren zur Herstellung von bromierten Alkoxy-substituierten Metall-Phthalocyaninen, insbesondere Kupfer- und Palladium-Phthalocyaninen.

Substituierte Phthalocyanine stellen eine wichtige Klasse von Farbstoffen für die optische Informationsaufzeichnung dar, da sie bei entsprechender peripherer Substitution, abhängig vom zentralen Metallatom, hohe NIR Absorptionen im Bereich von 700 nm bis 900 nm aufweisen.

Die Verwendung von Phthalocyanin-Farbstoffen, die im nahen Infrarotbereich (NIR-Bereich) Strahlung absorbieren, zur Aufzeichnung von Informationen in WORM-Systemen (write once read many), ist seit längerem bekannt und zum Beispiel von M. Emmelius in Angewandte Chemie, Heft 11, Seiten 1475-1502 (1989) beschrieben. Durch die Laserbestrahlung in solchen Aufzeichnungsmaterialien kann die für die Aufzeichnung von Informationen in Form von Bits notwendige Änderung der Absorption durch physikalische Veränderungen (zum Beispiel durch Sublimation oder Diffusion) oder durch chemische Veränderungen (zum Beispiel Photochromie, Isomerisierungen oder thermische Zersetzung) erzielt werden.

Metallkomplexe, insbesondere Palladium- und Kupferkomplexe von Alkoxy-substituierten, bromierten Phthalocyaninen sind ebenfalls bekannt und in der EP-A-513 370 sowie in der EP-A-519 419 beschrieben. Es handelt sich dabei um in organischen Lösungsmitteln lösliche, halogenierte, mit Alkoxy tetrasubstituierte Phthalocyanine, deren Alkoxy Gruppen sterisch anspruchsvolle Reste enthalten. Die Absorptionsmaxima der dort beschriebenen Verbindungen liegen bei ca. 700 nm bis ca. 730 nm und weisen einen molaren Extinktionskoeffizienten ε von >100 000 l·mol⁻¹·cm⁻¹ auf. Durch diese Eigenschaften kann man bei damit hergestellten optischen Speicherplatten einen ausreichend hohen Brechungsindex bei 780 nm und eine gute Empfindlichkeit erreichen.

Nach dem in der EP-A-513 370 und EP-A-519 419 beschriebenen Verfahren zur Herstellung dieser Verbindungen geht man von Alkoxy-substituierten Phthalocyaninen aus und bromiert diese in einem organischen Lösungsmittel, das mit Wasser nicht mischbar ist und bevorzugt aus der Gruppe, bestehend aus gesättigten Kohlenwasserstoffen, Ethern und halogenierten Kohlenwasserstoffen, ausgewählt wird.

Die dazu als Edukte benötigten α- oder β-Alkoxy substituierten Phthalocyanine selbst können nach ebenfalls bekannten Methoden hergestellt werden, zum Beispiel wie in Nouveau Journal de Chimie, Vol. 6, Seiten 653-658 (1982) und in der EP-A-492 508 beschrieben. Bei diesen bekannten Verfahren werden in der Regel jeweils verschiedene Stellungsisomere erhalten, die mehr oder weniger gute Löslichkeiten in organischen Lösungsmitteln aufweisen. Grundsätzlich eignen sich alle Isomerengemische von α- oder β-Alkoxy substituierten Phthalocyaninen zur Bromierung nach dem vorliegenden Verfahren. Bevorzugt sind jedoch überwiegend aus den gut löslichen Stellungsisomeren bestehende Isomerengemische, welche in hoher Ausbeute hergestellt werden können, falls man Alkoxysubstituierte Phthalodinitrile in Gegenwart von Nitrobenzol, Nitrotoluol oder Nitroxylol und mindestens einer equimolaren Menge Harnstoff, bezogen auf die eingesetzte Menge Phthalodinitril, umsetzt.

Bromierte Tetraalkoxymetallphthalocyanine, insbesondere die Kupfer- oder Palladium-Verbindungen, stellen selbst wichtige und wirtschaftliche Farbstoffe für die optische Informationsaufzeichnung dar. Dazu können sie als wichtige Zwischenprodukte dienen, in welchen zum Beispiel anschliessend zusätzliche phosphorhaltige Substituenten eingeführt werden können, wodurch sich die Polarität der Verbindungen und damit ihre Löslichkeit auf die verschiedensten Lösungsmittel anpassen lässt.

Eine hohe Ausbeute des Bromierungsschrittes ist für eine wirtschaftliche Herstellung dieser Verbindungen von grosser Wichtigkeit.

Da bei der Reaktion mehrfache Bromierungen in verschiedenen Positionen des peripheren aromatischen Kohlenstoffgerüsts auftreten, kommt der Reproduzierbarkeit und Selektivität der Reaktion ebenfalls eine wichtige Bedeutung zu. Das Reaktionsprodukt muss bezüglich Bromierungsgrad und -positionen, molarem Extinktionskoeffizient und Absorptionsspektrum (λₘₐₓ) gut reproduzierbar hergestellt werden können. Entstehende Nebenprodukte sollen in möglichst geringer Menge anfallen und sich leicht abtrennen lassen, so dass aufwendige Reinigungsverfahren, wie zum Beispiel chromatographische Verfahren, nicht notwendig sind. Bei den unerwünschten Nebenprodukten kann es sich beispielsweise um die von F. H. Moser und A. L. Thomas, Phthalocyanine Compounds, S. 54-59 (Reinhold Publishing Corporation, New York 1963), erwähnten Oxidationsprodukte handeln.

Tetraalkoxy-Palladiumphthalocyanine können zwar in guter Ausbeute in bekannten Lösungsmitteln wie 1,1,2-Trichlorethan bromiert werden, jedoch beobachtet man bei Tetraalkoxy-Kupferphthalocyaninen insbesondere in 1,1,2-Trichlorethan eine nahezu vollständige, unerwartete Zerstörung des Phthalocyaningerüsts.

Es wurde nun überraschend gefunden, dass man bromierte Tetraalkoxy-kupferphthalocyanine in hoher Ausbeute und mit sehr gut reproduzierbarem Substitutionsgrad erhält, wenn man die entsprechenden Tetraalkoxy-kupferphthalocyanine in einem mit Wasser im wesentlichen nicht mischbaren halogenierten aromatischen Lösungsmittel in Gegenwart einer zweiten, wässrigen Phase bromiert. Die Ausbeuten liegen erheblich über denen, die erreicht werden, wenn das Verfahren mit den in EP-A-513 370 oder EP-A-519 419 beschriebenen Lösungsmitteln durchgeführt wird.

Die wässerige Phase ist zweckmässig während der ganzen Dauer der Bromierungsreaktion vorhanden; sie kann aber auch periodisch zugefügt und wieder entfernt werden. Bevorzugt wird eine gute Durchmischung beider Phasen gewährleistet, zum Beispiel durch effiziente Rührung des Reaktionsgemisches oder Verdüsung einer Phase in die andere.

Ganz überraschend bekommt man sogar noch bessere Ausbeuten und Selektivitäten, wenn man der wässerigen Phase ein Reduktionsmittel zusetzt. Bevorzugt wird das Reduktionsmittel dabei nicht auf einmal bei Beginn der Bromierung zugesetzt, sondern so zudosiert, dass dessen Konzentration gerade noch reduzierend wirkt, oder besonders bevorzugt in Portionen unter Unterbrechung der Bromierung zugegeben. Dies kann beispielsweise auch derart geschehen, dass man zunächst reines Wasser als wässerige Phase benützt, dann zuweilen die Zufuhr von Brom unterbricht und die Wasserphase durch eine reduzierende wässerige Lösung ersetzt, und nach erneutem Ersatz der ganz oder teilweise verbrauchten reduzierenden wässerigen Lösung durch Wasser mit der Zufuhr von Brom fortfährt. Dieses Zyklus kann mehrmals wiederholt werden.

Bromierte Tetraalkoxy-palladiumphthalocyanine werden nach dem gleichen Verfahren ebenfalls in hoher Ausbeute und mit sehr gut reproduzierbarem Substitutionsgrad aus Tetraalkoxy-palladiumphthalocyaninen erhalten.

Das gleiche Verfahren kann ebenfalls zur Bromierung von Tetraalkoxy-phthalocyaninen von Zn(II), Sn(II), Ni(II), Co(II), Pb(II), Mn(O) oder V(O) verwendet werden.

Ein Gegenstand der Erfindung ist daher ein Verfahren zur Herstellung eines Stellungsisomerengemisches bromierter Tetraalkoxy-metallphthalocyanine der Formel I, 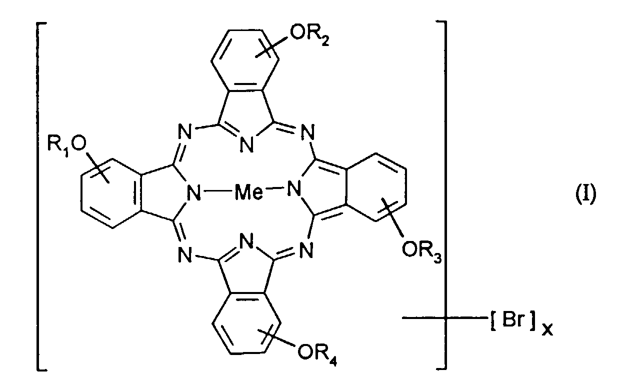 wobei
Me für Cu(II), Pd(II), Zn(II), Sn(II), Ni(II), Co(II), Pb(II), Mn(O) oder V(O) steht,
x eine Zahl von 1 bis 5 bedeutet, und
R₁ bis R₄ unabhängig voneinander lineare oder verzweigte C₁-C₁₆-Alkyl-, C₃-C₁₆-Alkenyl- oder C₃-C₁₆-Alkinylreste sind, die unsubstituiert oder mit C₁-C₁₂-Alkoxy, -CN, NO₂, Halogen, -OH, Phenyl, Cyanophenyl, Nitrophenyl, Halogenphenyl, Hydroxyphenyl oder (C₁-C₁₂-Alkoxy)phenyl substituiert sind,
durch Umsetzung eines Tetraalkoxy-Metallphthalocyanins der Formel II 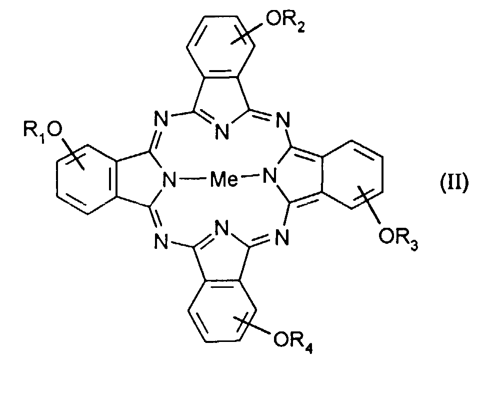 mit Brom, dadurch gekennzeichnet, dass die Reaktion in einem mit Wasser im wesentlichen nicht mischbaren halogenierten aromatischen Lösungsmittel in Gegenwart einer zweiten, wässrigen Phase durchgeführt wird.

In den Formeln I und II steht Me bevorzugt für Kupfer oder Palladium, besonders bevorzugt für Kupfer. Die Phthalocyanine der Formel I können an allen 4 Phenylringen des peripheren aromatischen Kohlenstoffgerüsts mit Brom substituiert sein. Bevorzugt sind mindestens 2 Phenylringe des peripheren aromatischen Kohlenstoffgerüsts mit Brom substituiert. Bevorzugt ist das periphere aromatische Kohlenstoffgerüst mit 1 bis 4 Brom substituiert.

Beispiele für lineare oder verzweigte C₁-C₁₆-Alkylreste sind z.B. Methyl, Ethyl sowie die verschiedenen Stellungsisomeren von Propyl, Butyl, Pentyl, Hexyl, Heptyl, Octyl, Nonyl, Decyl, Undecyl, Dodecyl, Tridecyl, Tetradecyl, Pentadecyl oder Hexadecyl.

Bevorzugt sind C₄-C₁₂-Alkylreste.

Beispiele für C₃-C₁₆-Alkenylreste sind Propenyl, Butenyl, Pentenyl, Hexenyl, Heptenyl, Octenyl, Nonenyl, Decenyl, Undecenyl, Dodecenyl, Tridecenyl, Tetradecenyl, Pentadecenyl oder Hexadecenyl mit ihren verschiedenen Stellungsisomeren.

Bevorzugt sind C₄-C₁₂-Alkenylreste.

Beispiele für C₃-C₁₆-Alkinylreste sind Propinyl, Butinyl, Pentinyl, Hexinyl, Heptinyl, Octinyl, Noninyl, Decinyl, Undecinyl, Dodecinyl, Tridecinyl, Tetradecinyl, Pentadecinyl oder Hexadecinyl mit ihren verschiedenen Stellungsisomeren.

Bevorzugt sind C₄-C₁₂-Alkinylreste.

Bevorzugt sind die Alkyl-, Alkenyl- und Alkinylreste verzweigt.

Halogen steht zum Beispiel für Fluor, Brom, Chlor oder Jod.

C₁-C₁₂-Alkoxy bedeutet zum Beispiel Methoxy, Ethoxy sowie die verschiedenen Stellungsisomeren von Propoxy, Butoxy, Pentoxy, Hexyloxy, Heptyloxy, Octyloxy, Nonyloxy, Decyloxy, Undecyloxy oder Dodecyloxy.

Bevorzugt sind C₁-C₈-Alkoxy.

Bevorzugt bedeuten R₁ bis R₄ lineare oder verzweigte C₁-C₁₆-Alkylreste, die unsubstituiert oder mit C₁-C₁₂-Alkoxy, -CN, NO₂, Halogen, -OH, Phenyl, Cyanophenyl, Nitrophenyl, Halogenphenyl, Hydroxyphenyl oder (C₁-C₁₂-Alkoxy)phenyl substituiert sind.

In einer besonders bevorzugten Untergruppe bedeuten R₁ bis R₄ lineare oder verzweigte C₄-C₁₂-Alkylreste, die unsubstituiert oder mit C₁-C₁₂-Alkoxy, -CN, NO₂, Halogen, -OH, Phenyl, Cyanophenyl, Nitrophenyl, Halogenphenyl, Hydroxyphenyl oder (C₁-C₁₂-Alkoxy)phenyl substituiert sind.

Ganz besonders bevorzugt sind R₁ bis R₄ unsubstituierte lineare oder verzweigte C₄-C₁₂-Alkylreste, insbesondere 2,4-Dimethyl-3-pentyl.

Die Substituenten -OR₁ bis -OR₄ können sowohl in β- als auch in α-Stellung stehen, bevorzugt stehen alle Substituenten -OR₁ bis -OR₄ in α-Stellung.

Im Falle der α-oder β-Substitution liegen bei identischen R₁ bis R₄ im wesentlichen jeweils 4 Stellungsisomere vor, die sich durch ihre Löslichkeit unterscheiden. Sind R₁ bis R₄ verschieden, so erhöht sich die Anzahl möglicher Verbindungen im Produktgemisch.

Das vorliegende Verfahren eignet sich insbesondere vorzüglich zur Herstellung bromierter Verbindungen der Formel I, worin R₁ bis R₄ identisch sind, vornehmlich zur Herstellung bromierter Verbindungen der Formel I, worin R₁ bis R₄ identisch sind und die Substituenten -OR₁ bis -OR₄ jeweils in α-Stellung stehen.

Bevorzugte halogenhaltige aromatische Lösungsmittel sind 1-Bromnapthalin, 1-Chlornaphthalin, 1-Chlornaphthalin / 2-Chlornaphthalin, eines der Stellungsisomeren von Bromtoluol, Chlortoluol oder Dichlortoluol, eines der Stellungsisomeren von Dibrombenzol oder Dichlorbenzol, Brombenzol oder Chlorbenzol.

Besonders bevorzugtes halogenhaltiges aromatisches Lösungsmittel ist Chlorbenzol.

Eine bevorzugte Verfahrensdurchführung ergibt sich, wenn das Verfahren so durchgeführt wird, dass Brom im gleichen halogenhaltigen aromatischen Lösungsmittel wie die Verbindungen der Formel II gelöst wird und als Lösung der Reaktion zudosiert wird.

Bevorzugt ist das Gewichtsverhältnis von Brom zu halogenhaltigem aromatischem Lösungsmittel 1:10 bis 10:1, besonders bevorzugt 1:5 bis 5:1.

Das Verfahren wird vorzugsweise unter Schutzgasatmosphäre, beispielsweise unter Stickstoff oder Argon, durchgeführt.

Bevorzugt beträgt das das Gewichtsverhältnis von halogenhaltigem aromatischen Lösungsmittel zu Wasser 10:1 bis 1:10, besonders bevorzugt 4:1 bis 1:1.

Die Reaktionstemperatur beträgt bevorzugt 20°C bis 150°C, besonders bevorzugt 30°C bis 90°C.

Bevorzugt wird das Verfahren bei Normaldruck durchgeführt.

Enthält die wässerige Phase gegebenenfalls ein Reduktionsmittel, so kann es sich dabei beispielsweise um ein wasserlösliches organisches oder anorganisches Reduktionsmittel handeln. Wasserlösliche organische Reduktionsmittel sind zum Beispiel Formaldehyd, Hydrochinon oder Ameisen- und Oxalsäure oder deren Salze; wasserlösliche anorganische Reduktionsmittel sind zum Beispiel Phosphor(III)-, Eisen(II)-, Schwefel(IV)- oder Stickstoff-Verbindungen, wie Triethylphosphit, Eisensulfat-Heptahydrat, Natriumsulfit, Kaliumsulfit, Natriumpyrosulfit, Kaliumpyrosulfit, Natriumthiosulfat, Natriumdithionit, Natriumdithionat, Kaliumdithionat, Natriumjodid, Kaliumjodid, Hydroxylamin oder Hydrazin.

Bevorzugte Reduktionsmittel sind Alkalimetall-sulfite, -pyrosulfite und -thiosulfate, besonders bevorzugt sind wässrige Natriumsulfit- und Kaliumsulfitlösungen.

Die nachfolgenden Beispiele verdeutlichen die Erfindung.

### Herstellung der Zwischenverbindungen

### Beispiel A1: Tetra-(α-2,4-dimethyl-3-pentyloxy)-kupferphthalocyanin (Isomerengemisch)

In 410 ml Nitrobenzol werden 100,0 g (0,41 Mol) 3-(2,4-Dimethyl-3-pentyloxy)-phthalodinitril, 14,0 g (0,1 Mol) Kupfer(II)chlorid, 49,6 g (0,82 Mol) Harnstoff und 2,0 g (2 Gew.%) Ammoniummolybdat vorgelegt und unter Rühren und Argonatmosphäre auf 160°C erwärmt und anschliessend 5 Std bei dieser Temperatur gerührt. Anschliessend wird auf RT abgekühlt, mit Toluol verdünnt und durch ein Filterhilfsmittel filtriert. Das Filtrat wird bei 10°C/10⁻¹mbar vollständig eingedampft. Der Rückstand wird in 1 l Toluol gelöst und mit Toluol als Laufmittel über 600 g Kieselgel filtriert. Das Filtrat wird eingedampft, der Rückstand in 1,5 l Methanol aufgerührt, filtriert, mit Methanol gewaschen und über Nacht bei 60°C/165 mbar getrocknet. Man erhält 99,5 g (94% d.Th.) eines grün-blauen Feststoffes mit einem λₘₐₓ von 712 nm (ε = 197'680 l·mol⁻¹·cm⁻¹) in N-Methylpyrrolidon (NMP). Eine Dünnschichtchromatographie zeigt, dass die Isomeren I, II und III in einem Verhältnis von 5 zu 33 zu 62 vorliegen.

### Beispiel A2: Tetra-(α-2,4-dimethyl-3-pentyloxy)-kupferphthalocyanin (Isomerengemisch)

In einem 1 l-Dreihalskolben, versehen mit Rückflusskühler, Magnetrührer, Thermometer und Stickstoffüberleitung, werden 50 g (0,206 Mol) 3-(2,4-Dimethyl-3-pentyloxy)-phthalodinitril, 7,0 g (51,7 mMol) Kupfer(II)chlorid, 24,8 g (0,413 Mol) Harnstoff, 1,0 g (2 Gew.%) Ammoniummolybdat und 200 ml Nitrobenzol vorgelegt, und unter Inertgasatmosphäre und Rühren 5x Std bei 160°C gerührt. Anschliessend wird auf RT abgekühlt, das Reaktionsgemisch mit 200 ml Toluol verdünnt, über ein Filterhilfsmittel filtriert und 5x mit je 100 ml Toluol nachgewaschen. Das Filtrat wird mit Toluol über 500 g Kieselgel filtriert und die grünen Fraktionen bei 110°C am Wasserstrahlvakuum möglichst vollständig eingedampft. Man erhält 72,1 g eines grünen Harzes, welches noch wenig Nitrobenzol enthält und ein λₘₐₓ von 712 nm in NMP aufweist.

### Beispiel A3: Tetra-(α-2,4-dimethyl-3-pentyloxy)-palladiumphthalocyanin (Isomerengemisch)

In 200 ml Nitrobenzol werden 50 g (206 mMol) 3-(2,4-Dimethyl-3-pentyloxy)phthalodinitril, 9,1 g (51,7 mMol) wasserfreies Palladiumchlorid, 24,8 g (413 mMol) Harnstoff und 1 g (2 Gew.%) Ammoniummolybdat vorgelegt und unter Rühren und Argonatmosphäre auf 160°C erwärmt. Anschliessend wird 4 Std bei dieser Temperatur gerührt, danach auf RT abgekühlt, mit Toluol verdünnt und über ein Filterhilfsmittel filtriert. Das Filtrat wird bei 100°C/10⁻¹mbar vollständig eingedampft. Der Rückstand wird in 400 ml Toluol aufgenommen und mit Toluol als Laufmittel über 500 g Kieselgel filtriert. Die Toluolphase wird auf 250 ml eingedampft und anschliessend auf 1,5 l Methanol getropft. Der Niederschlag wird abfiltriert und zweimal mit 100 ml Methanol gewaschen. Danach wird 12 Std bei 60°C/165 mbar getrocknet. Man erhält 32,5 g (59% d. Th.) eines grün-blauen Feststoffes mit einem λₘₐₓ von 702 nm (ε = 215'190 l·mol⁻¹·cm⁻¹) in NMP. Das NMR zeigt, dass die Isomeren I, II und III in einem Verhältnis von 5 zu 53 zu 42 vorliegen.

### Herstellung der Endprodukte

### Beispiel B1: bromiertes Tetra-(α-2,4-dimethyl-3-pentyloxy)-kupferphthalocyanin

2 g (1,94 mMol) Tetra-(α-2,4-dimethyl-3-pentyloxy)-kupfer-phthalocyanin, hergestellt nach Beispiel A1, werden in 20 g Chlorbenzol und 10 g Wasser vorgelegt. Unter Rühren und Argonatmosphäre werden bei 40°C innerhalb von 10 Minuten 0,96 g (6,0 mMol) Brom in 1 g Chlorbenzol zugetropft und anschliessend 1 Stunde bei 60°C gerührt. Das Reaktionsgemisch wird abgekühlt, mit 50 ml Chlorbenzol verdünnt, 1× mit 50 ml wässriger 3%iger NaHSO₃ gewaschen, über MgSO₄ getrocknet, filtriert und eingedampft. Der Rückstand wird in Toluol gelöst und mit Toluol über 50 g Kieselgel filtriert. Das Filtrat wird vollständig eingedampft und über Nacht bei 60°C/165mbar getrocknet. Man erhält 2,0 g (84,7% d.Th.) eines grünen Pulvers mit einem λₘₐₓ von 727 nm in NMP (ε = 145'584 l·mol⁻¹·cm⁻¹) und einem Bromgehalt von 17,2%.

### Beispiel B2: bromiertes Tetra-(α-2,4-dimethyl-3-pentyloxy)-kupferphthalocyanin

Das Rohprodukt aus Beispiel A2 wird in einem 2,5 l-Mehrhalskolben, versehen mit Ankerrührer, Thermometer, Rückflusskühler und Tropftrichter, in 735 g Chlorbenzol und 362 g Wasser vorgelegt und unter Rühren auf 40°C erwärmt. Danach werden innerhalb von 30 Min 7,81 g (48,9 mMol) Brom in 20 g Chlorbenzol zugetropft und 1 Std bei derselben Temperatur gerührt. Die Lösung weist ein λₘₐₓ von 715 nm in NMP auf. Es werden 300 ml 3%ige NaHSO₃-Lösung zugegeben und 5 Min gerührt. Die wässerige Phase wird abgesaugt, dann werden 300 ml Wasser zugegeben und nach kurzer Rührung erneut abgesaugt. Anschliessend werden wieder 362 g Wasser zugegeben und wie oben beschrieben 7,81 g (48,9 mMol) Brom zugetropft. Diese Operationen werden wiederholt, bis total 25,77 g (0,161 Mol) Brom zur Reaktion gebracht worden sind und die Lösung ein λₘₐₓ von 722 nm in NMP aufweist. Die Lösung wird abgekühlt, die Phasen getrennt und die organische Phase über Magnesiumsulfat getrocknet. Die Lösung wird mit Toluol über 300 g Kieselgel filtriert und die grüne Fraktion bei 110°C am Wasserstrahlvakuum eingedampft. Der Rückstand wird in 100 ml Toluol aufgenommen und unter Rühren auf 2 I Methanol getropft. Der Niederschlag wird abfiltriert, 3 mal mit je 100 ml Methanol gewaschen und über Nacht bei 60°C/165mbar getrocknet.
Man erhält 44,22 g (70,6% d.Th. über 2 Stufen) eines grünen Feststoffes mit einem λₘₐₓ von 722 nm in NMP (ε= 172'510 l·mol⁻¹·cm⁻¹) und einem Bromgehalt von 15,18%.

### Beispiel B3: bromiertes Tetra-(α-2,4-dimethyl-3-pentyloxy)-palladiumphthalocyanin

10 g (9,30 mMol) Tetra-(α-2,4-dimethyl-3-pentyloxy)-palladiumphthalocyanin, hergestellt nach Beispiel A3, werden in einem 500 ml-Mehrhalskolben, versehen mt Ankerrührer, Thermometer, Rückflusskühler und Tropftrichter, in 100 g Chlorbenzol und 50 g Wasser vorgelegt und unter Rühren auf 40°C erwärmt. Danach werden innerhalb von 10 Min 7,4 g (46,48 mMol) Brom in 2 g Chlorbenzol zugetropft und 1 Std bei 60°C gerührt. Die Lösung wird abgekühlt, mit 100 ml Chlorbenzol verdünnt und mit 50 ml 3%iger NaHSO₃-Lösung gewaschen. Die organische Phase wird über Magnesiumsulfat getrocknet und eingedampft. Der grüne Rückstand wird in Toluol gelöst, mit Toluol über 200 g Kieselgel filtriert und die grüne Fraktion am Wasserstrahlvakuum bis auf 80 ml eingedampft. Diese Lösung wird unter Rühren auf 700 ml Methanol getropft. Der Niederschlag wird abfiltriert, 3× mit je 50 ml Methanol gewaschen und über Nacht bei 60°C/165mbar getrocknet.
Man erhält 11,6 g (88,1% d.Th.) eines grünen Feststoffes mit einem λₘₐₓ von 724 nm in NMP (ε = 163'210 l·mol⁻¹·cm⁻¹) und einem Bromgehalt von 24,24%.

### Vergleichsbeispiel

### Vergleichsbeispiel V1 (zu Beispiel B1):

Bromierung von Tetra-(α-2,4-dimethyl-3-pentyloxy)-kupferphthalocyanin

2 g (1,94 mMol) Tetra-(α-2,4-dimethyl-3-pentyloxy)-kupferphthalocyanin, hergestellt nach Beispiel A1, werden in 20 g 1,1,2-Trichlorethan und 11 g Wasser vorgelegt. Unter Rühren und Argonatmosphäre werden bei 40°C innerhalb von 25 Minuten 0,82 g (5,11 mMol) Brom in 2 g 1,1,2-Trichlorethan zugetropft. Das Reaktionsgemisch zersetzt sich zu einer braunen Lösung. Mit Dünnschichtchromatographie und UV/VIS Spektroskopie können weder Edukt noch erwünschtes Produkt gemäss Beispiele B1 oder B2 nachgewiesen werden.

## Patentansprüche

1. Verfahren zur Herstellung eines Stellungsisomerengemisches bromierter Tetraalkoxy-metallphthalocyanine der Formel I, 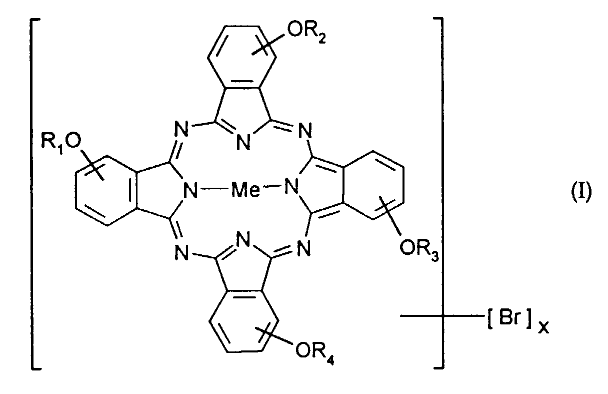 wobei
Me für Cu(II), Pd(II), Zn(II), Sn(II), Ni(II), Co(II), Pb(II), Mn(O) oder V(O) steht,
x eine Zahl von 1 bis 5 bedeutet, und
R₁ bis R₄ unabhängig voneinander lineare oder verzweigte C₁-C₁₆-Alkyl-, C₃-C₁₆-Alkenyl-oder C₃-C₁₆-Alkinylreste sind, die unsubstituiert oder mit C₁-C₁₂-Alkoxy, -CN, NO₂, Halogen, -OH, Phenyl, Cyanophenyl, Nitrophenyl, Halogenphenyl, Hydroxyphenyl oder (C₁-C₁₂-Alkoxy)phenyl substituiert sind,
durch Umsetzung eines Tetraalkoxy-Metallphthalocyanins der Formel II 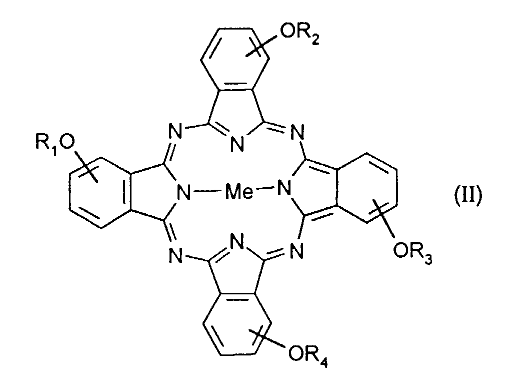 mit Brom, dadurch gekennzeichnet, dass die Reaktion in einem mit Wasser im wesentlichen nicht mischbaren halogenierten aromatischen Lösungsmittel in Gegenwart einer zweiten, wässrigen Phase durchgeführt wird.

2. Verfahren nach Anspruch 1, worin R₁ bis R₄ lineare oder verzweigte C₁-C₁₆-Alkylreste, insbesondere lineare oder verzweigte C₄-C₁₂-Alkylreste, die unsubstituiert oder mit C₁-C₁₂-Alkoxy, -CN, NO₂, Halogen, -OH, Phenyl, Cyanophenyl, Nitrophenyl, Halogenphenyl, Hydroxyphenyl oder (C₁-C₁₂-Alkoxy)phenyl substituiert sind, bedeuten, bevorzugt worin R₁ bis R₄ unsubstituierte lineare oder verzweigte C₄-C₁₂-Alkylreste bedeuten, besonders bevorzugt worin R₁ bis R₄ identisch sind und insbesondere 2,4-Dimethyl-3-pentyl bedeuten.

3. Verfahren nach Anspruch 1, worin die Substituenten -OR₁ bis -OR₄ in α-Stellung stehen.

4. Verfahren nach Anspruch 1, worin mindestens 2 Phenylringe des peripheren aromatischen Kohlenstoffgerüsts mit Brom substituiert sind.

5. Verfahren nach Anspruch 1, worin das periphere aromatische Kohlenstoffgerüst mit 1 bis 4 Brom substituiert ist.

6. Verfahren nach Anspruch 1, worin Me Kupfer oder Palladium, bevorzugt worin Me Kupfer bedeutet.

7. Verfahren nach Anspruch 1, worin das halogenhaltige aromatische Lösungsmittel 1-Bromnapthalin, 1-Chlornaphthalin, 1-Chlornaphthalin 2-Chlornaphthalin, eines der Stellungsisomeren von Bromtoluol, Chlortoluol oder Dichlortoluol, eines der Stellungsisomeren von Dibrombenzol oder Dichlorbenzol, Brombenzol oder Chlorbenzol, bevorzugt Chlorbenzol ist.

8. Verfahren nach Anspruch 1, worin Brom im gleichen halogenhaltigen aromatischen Lösungsmittel wie die Verbindungen der Formel II gelöst wird und als Lösung der Reaktion zudosiert wird.

9. Verfahren nach Anspruch 8, worin das Gewichtsverhältnis von Brom zu halogenhaltigem aromatischen Lösungsmittel 1:10 bis 10:1, bevorzugt 1:5 bis 5:1 beträgt.

10. Verfahren nach Anspruch 1, worin die Reaktion unter Schutzgasatmosphäre durchgeführt wird.

11. Verfahren nach Anspruch 1, worin das Gewichtsverhältnis von halogenhaltigem aromatischen Lösungsmittel zu Wasser 10:1 bis 1:10, bevorzugt 4:1 bis 1:1 beträgt.

12. Verfahren nach Anspruch 1, worin die Reaktion bei einer Temperatur von 20°C bis 150°C, bevorzugt bei 30°C bis 90°C durchgeführt wird.

13. Verfahren nach Anspruch 1, worin bei Normaldruck gearbeitet wird.

14. Verfahren nach Anspruch 1, worin beide Phasen gut durchmischt werden.

15. Verfahren nach Anspruch 1, worin die wässerige Phase ein Reduktionsmittel, bevorzugt ein Alkalimetall-sulfit, -pyrosulfit oder -thiosulfat, enthält.

16. Verfahren nach Anspruch 15, worin das zugesetzte Reduktionsmittel in Portionen unter Unterbrechung der Bromierung zugegeben wird.

## Claims

1. A process for the preparation of a mixture of positionally isomeric brominated tetraalkoxymetal phthalocyanines of the formula **I** 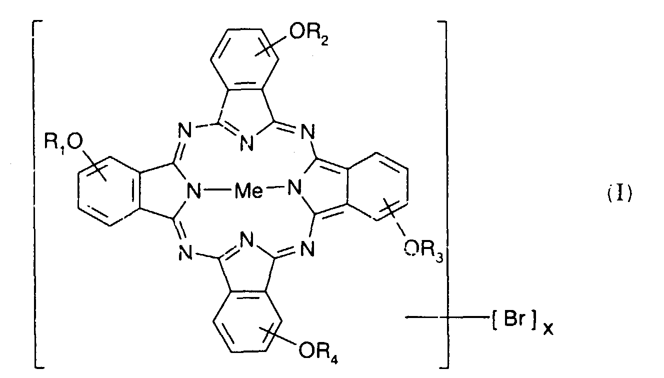 where
Me is Cu(II), Pd(II), Zn(II), Sn(II), Ni(II), Co(II), Pb(II), Mn(O) or V(O),
x is a number from 1 to 5, and
R₁ to R₄, independently of one another, are linear or branched C₁-C₁₆alkyl, C₃-C₁₆alkenyl or C₃-C₁₆alkynyl radicals which are unsubstituted or substituted by C₁-C₁₂alkoxy, -CN, NO_{2,} halogen, -OH, phenyl, cyanophenyl, nitrophenyl, halophenyl, hydroxyphenyl or (C₁-C₁₂alkoxy)phenyl,
by reacting a tetraalkoxymetal phthalocyanine of the formula **II** 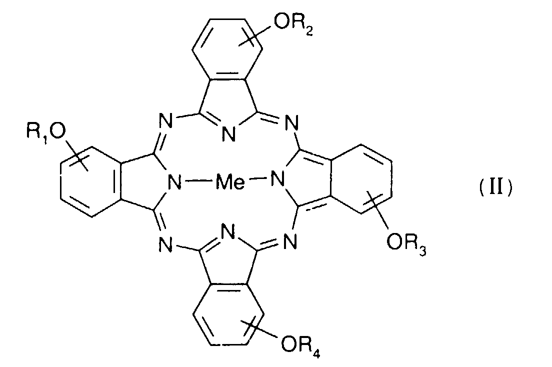 with bromine, which comprises carrying out the reaction in a halogenated, essentially water-immiscible aromatic solvent in the presence of a second, aqueous phase.

2. A process according to claim 1, in which R₁ to R₄ are linear or branched C₁-C₁₆alkyl radicals, linear or branched C₄-C₁₂alkyl radicals, which are unsubstituted or substituted by C₁-C₁₂alkoxy, -CN, NO₂, halogen, -OH, phenyl, cyanophenyl, nitrophenyl, halophenyl, hydroxyphenyl or (C₁-C₁₂alkoxy)phenyl, preferably in which R₁ to R₄ are unsubstituted linear or branched C₄-C₁₂alkyl radicals, particularly preferably in which R₁ to R₄ are identical and in particular are 2,4-dimethyl-3-pentyl.

3. A process according to claim 1, in which the substituents -OR₁ to -OR₄ are in the α-position.

4. A process according to claim 1, in which at least 2 phenyl rings of the peripheral aromatic carbon skeleton are substituted by bromine.

5. A process according to claim 1, in which the peripheral aromatic carbon skeleton is substituted by from 1 to 4 bromine atoms.

6. A process according to claim 1, in which Me is copper or palladium, preferably in which Me is copper.

7. A process according to claim 1, in which the halogen-containing aromatic solvent is 1-bromonaphthalene, 1-chloronaphthalene, 1-chloronaphthalene/2-chloronaphthalene, one of the positional isomers of bromotoluene, chlorotoluene or dichlorotoluene, one of the positional isomers of dibromobenzene or dichlorobenzene, bromobenzene or chlorobenzene, preferably chlorobenzene.

8. A process according to claim 1, in which bromine is dissolved in the same halogen-containing aromatic solvent as the compounds of the formula **II**, and is metered into the reaction as a solution.

9. A process according to claim 8, in which the bromine:halogen-containing aromatic solvent weight ratio is from 1:10 to 10:1 from 1:5 to 5:1.

10. A process according to claim 1, in which the reaction is carried out under a protective-gas atmosphere.

11. A process according to claim 1, in which the halogen-containing aromatic solvent:water weight ratio is from 10:1 to 1:10, preferably from 4:1 to 1:1.

12. A process according to claim 1, in which the reaction is carried out at a temperature of from 20°C to 150°C, preferably at from 30°C to 90°C.

13. A process according to claim 1, which is carried out at atmospheric pressure.

14. A process according to claim 1, in which the two phases are mixed well.

15. A process according to claim 1, in which the aqueous phase contains a reducing agent, preferably an alkali metal sulfite, pyrosulfite or thiosulfite.

16. A process according to claim 15, in which the reducing agent is added in portions with interruption of the bromination.

## Revendications

1. Procédé de préparation d'une mélange d'isomères de position de tétraalkoxyphtalocyanines métalliques bromées de formule I 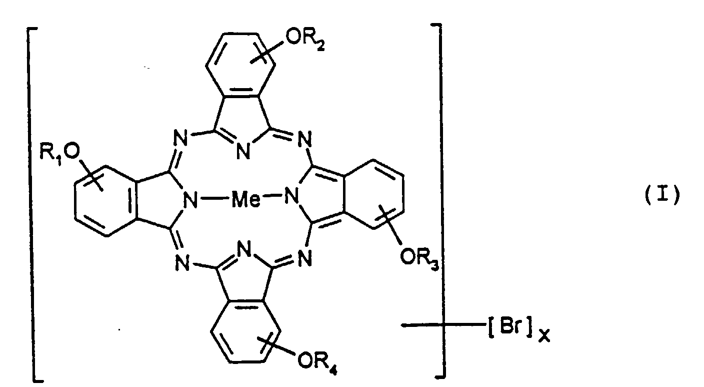 où
Me représente Cu(II), Pd(II), Zn(II), Sn(II), Ni(II), Co(II), Pb(II), Mn(O) ou V(O),
x est un nombre de 1 à 5, et
R₁ à R₄ représentent, indépendamment les uns des autres, des restes alkyle en C₁-C₁₆, alcényle en C₃-C₁₆ ou alcynyle en C₃-C₁₆ à chaîne droite ou ramifiée, non substitués ou substitués par des substituants alkoxy en C₁-C₁₂, -CN, NO₂, halogène, -OH, phényle, cyanophényle, nitrophényle, halophényle, hydroxyphényle ou (alkoxy en C₁-C₁₂)phényle,
par réaction d'une tétraalkoxyphtalocyanine métallique de formule II 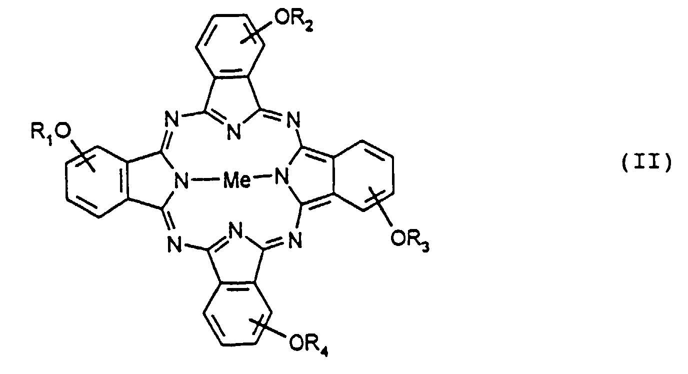 avec du brome, caractérisé en ce que l'on met en oeuvre la réaction dans un solvant aromatique halogéné, essentiellement non miscible à l'eau, en présence d'une seconde phase, aqueuse.

2. Procédé selon la revendication 1, où R₁ à R₄ représentent, indépendamment les uns des autres, des restes alkyle en C₁-C₁₆ à chaîne droite ou ramifiée, en particulier à chaîne droite, ou des restes alkyle en C₄-C₁₂ ramifiés, lesquels sont non substitués ou substitués par des substituants alkoxy en C₁-C₁₂, -CN, NO₂, halogène, -OH, phényle, cyanophényle, nitrophényle, halophényle, hydroxyphényle ou (alkoxy en C₁-C₁₂)-phényle, de préférence dans lesquels R₁ à R₄ représentent des restes alkyle en C₄-C₁₂ à chaîne droite ou ramifiée non substitués, de manière particulièrement préférée R₁ à R₄ sont identiques et représentent en particulier 2,4-diméthyl-3-pentyle.

3. Procédé selon la revendication 1, où les substituants -OR₁ à -OR₄ se trouvent en position α.

4. Procédé selon la revendication 1, où au moins deux cycles phényle du squelette carboné aromatique périphérique sont substitués par du brome.

5. Procédé selon la revendication 1, où le squelette carboné aromatique périphérique est substitué par 1 à 4 atomes de brome.

6. Procédé selon la revendication 1, où Me représente le cuivre ou le palladium, de préférence dans lequel Me représente le cuivre.

7. Procédé selon la revendication 1, où le solvant aromatique halogéné est le 1-bromonaphtalène, le 1-chloronaphtalène, le 1-chloronaphtalène/2-chloronaphtalène, un des isomères de position de bromotoluène, de chlorotoluène ou de dichlorotoluène, un des isomères de position de dibromobenzène ou de dichlorobenzène, de bromobenzène ou de chlorobenzène.

8. Procédé selon la revendication 1, où le brome est dissout dans le même solvant aromatique halogéné que les composés de formule II et que l'on ajoute comme solution à la réaction.

9. Procédé selon la revendication 8, où le rapport pondéral du brome au solvant aromatique halogéné est de 1:10 à 10/1, de préférence de 1:5 à 5:1.

10. Procédé selon la revendication 1, où la réaction est mise en oeuvre sous une atmosphère de gaz protecteur.

11. Procédé selon la revendication 1, où le rapport pondéral du solvant artomatique halogéné à l'eau est de 10:1 à 1:10, de préférence de 4:1 à 1:1.

12. Procédé selon la revendication 1, où la réaction est mise en oeuvre à une température de 20°C à 150°C, de préférence de 30°C à 90°C.

13. Procédé selon la revendication 1, où on opère à la pression normale.

14. Procédé selon la revendication 1, où on mélange à fond les deux phases.

15. Procédé selon la revendication 1, où la phase aqueuse contient un agent réducteur, de préférence un sulfite, un pyrosulfite ou un thiosulfate de métaux alcalins.

16. Procédé selon la revendication 1, où on ajoute l'agent réducteur par portions en interrompant la bromation.
